# EUROPEAN PATENT APPLICATION

(11) **EP 2 037 038 A1**
(43) Date of publication of application: **18.03.2009**
(21) Application number: 08164169.8
(22) Date of filing: 11.09.2008
(51) Int. Cl.: D06H 1/02, G01N 21/88, G01N 33/36

(54) **Method for tracing defects on textiles**

(30) Priority: 14.09.2007 IT MI20071780
(71) Applicant: Mectex S.p.A., 22036 Erba CO (IT); Stazione Sperimentale per la Seta, 20133 Milano (IT)
(72) Inventor: Soffiantini, Paolo, 25121 MANERBIO (BRESCIA) (IT); Fassi, Aurelio, 22036 ERBA (COMO) (IT); Marcandalli, Bruno, 20069 VAPRIO D'ADDA (MILANO) (IT); Colonna, Gian Maria, 21055 GORLA MINORE (VARESE) (IT)
(74) Representative: Tansini, Elio Fabrizio

(57) **Abstract**

A method of tracing defects in textiles comprising the steps of detecting defects in a textile article of clothing and making marks thereon for identification of the detected defects. The marks are obtained by laying an identification or tracing substance on the textile product, which substance is substantially transparent to electromagnetic radiation having wavelengths included in the visible spectrum and opaque to electromagnetic radiation having wavelengths out of the visible spectrum, in particular in the infrared spectrum. The method comprises the step of detecting the marks on the textile product by means of a viewer sensitive to electromagnetic radiation having wavelengths to which the tracing substance is opaque. The tracing substance can also be fluorescent in the infrared spectrum.

## Description

The present invention relates to a method of tracing defects on textiles.

A large number of very diversified production steps, from the thread until the final product, are required in textiles; actually they go from the thread production to manufacture of the yarns, weaving, dyeing, printing and finish, until the final cutting and tailoring operations. In most cases these steps are carried out by different operators, usually located at great distances from each other, each of them being skilled in a specific sector.

Each individual operating step obviously and unavoidably involves the presence of defects that make it necessary to carry out continuous re-examinations on the part of the different operators, which will give rise to an important waste in terms of time (which is a factor of the greatest importance in the fashion field) and production rejects.

This is particularly prominent in the final steps of the production process, i.e. weaving, cut and tailoring. Therefore tracing and marking the defects present on textiles is a problem of the greatest importance.

At the present state of the art, at the end of working a weaver carries out a full examination of each individual roll of cloth, tracing the defects present thereon and manually marking them using a piece of chalk or adhesive labels.

Execution of this operation takes place by moving forward the roll on an illuminated screen, known in the technical field with the term "fabric inspection machine", while a qualified operator traces the different defects and marks them. The garment maker, on receiving the textile product, re-examines the supplied rolls of cloth, eliminates the portions that cannot be worked and carries out a new marking of the defects to make the rolls of cloth ready for the subsequent working operations, using sticks of chalk or mechanical systems, such as insertion of removable indication threads, which are referred to as "hornet stitch" in the technical field. The examination is quite necessary because the rolls of cloth are required to be submitted to cutting operations in accordance with the pattern corresponding to the initial project of the article of clothing. The garment maker therefore re-examines the cut lengths of cloth, manually rejects the lengths that cannot be used and begins making the true garment. The finished article is then sent to marketing after a further final re-examination aiming at verifying the presence of articles with defects which cannot be marketed or only marketed as second-best goods.

It is well apparent that the control operations constitute a very important part of the production process both in terms of costs and in terms of time.

The above described known-art methods of tracing defects however have some drawbacks. In fact, the used markers, such as adhesive labels or threads inserted by means of said particular "hornet stitch" can come off or be easily removed without leaving any trace, so that unsuitable parts of the textile product are worked and articles with defects are produced.

The marks made with a stick of chalk require a further cleaning operation to eliminate the remaining signs, otherwise they too could become the source of defects on the finished article. This cleaning operation is not always easy and does not always give the expected results of complete elimination.

In addition, during a final re-examination step before delivery of the finished articles to the customers, the said marks are required to be still present in order to be able to eliminate possible articles made by mistake, as pre-existent defects were present thereon. Due to the fact that in many cases the presence of defects may not be apparent when a quick examination even by qualified staff is carried out, this increases the possibility of putting faulty articles on the market.

In addition, the tracing methods adopted by the known art can give rise to doubts and consequent legal actions as regards who is to be held responsible for a non-correct previous information about the defect, because the marks can be removed even accidentally during the different working steps.

In this context, it is a technical task of the present invention to make available a method of tracing defects in textiles that is devoid of the above mentioned drawbacks.

Within the scope of this technical task, it is an aim of the present invention to propose a method of tracing defects in textiles that prevents possible disputes about a lack of information on the defects, due to causes that cannot be imputed to the person responsible for signalling said defects, such as transport of the textile products for example.

Another important aim is to make available a method of tracing defects in textiles in which the method itself does not give rise to defects in the textile product.

A still further aim of the invention is to make available a method of tracing defects in textiles that prevents unsuitable parts of the textile product from being erroneously worked.

The foregoing and still further aims that will become apparent in the following of the present specification are substantially achieved by a method of tracing defects in textiles comprising the steps set out in claim 1 and/or in one or more of the claims depending on said claim 1.

A preferred but not exclusive embodiment of a method of tracing defects in textiles in accordance with the present invention is now disclosed, by way of nonlimiting example. This description will be set forth hereinafter with reference to the accompanying drawings, in which:
- Fig. 1 shows the variation in the radiation absorption of a first substance to be used to the ends of the present invention, upon variation of the wavelength reproduced in the x-axes;
- Fig. 2 shows the variation in the radiation absorption of a second substance to be used to the ends of the present invention, upon variation of the wavelength reproduced in the x-axes ;
- Fig. 3 shows, the variation in the sensitivity to radiation of a digital video camera to be used to the ends of the present invention, upon variation of the wavelength reproduced in the x-axes;
- Fig. 4 shows the variation in emitting radiation from a radiant source to be used to the ends of the present invention, upon variation of the wavelength reproduced in the x-axes.

The method of the present invention comprises the steps of detecting defects in a textile product and making identification marks of the detected defects on said textile product.

Detection of the defects can be carried out by passing the fabric on an illuminated screen, of the type known in the technical field as, for example, "fabric inspection machine", while simultaneously a qualified operator traces the various defects and marks them.

Marking is carried out by laying an identification or tracing substance on the textile product, which substance is transparent to electromagnetic waves having wavelengths included in the visible spectrum and opaque to electromagnetic waves having wavelengths out of the visible spectrum.

It is to be pointed out that by the term "transparency" it is intended, in the context of the present invention, the property of the material of not interacting with electromagnetic radiation, enabling it to pass through the material without being submitted to important alterations.

As said, the identification or tracing substance is substantially transparent to the wavelengths in the visible spectrum, which means that the absorbed radiation to these wavelengths, included just as an indication between about 350 nm and about 700 nm, is negligible, i.e. it cannot be seen by the human eye.

The tracing substance on the contrary is substantially opaque to electromagnetic radiation with frequencies different from those of the visible spectrum, which means that it absorbs electromagnetic radiation with frequencies different from those of the visible spectrum.

This feature allows the fabric to be marked without this marking being visible "with the naked eye", and therefore without removal of marks possibly still present on the already made articles of manufacture being required.

In more detail, absorption of electromagnetic radiation at a wavelength out of the visible spectrum by the tracing substance is at least 2.5 times greater than absorption of electromagnetic radiation at wavelengths included in the visible spectrum. Preferably, the tracing substance is opaque to frequencies in the infrared spectrum alone, which as an indication have wavelengths in the range of 770 nm to 1200 nm.

In other words, the tracing substance absorbs electromagnetic radiation having a given wavelength included between 770 nm and 1200 nm, to an extend at least 2.5 times greater than it is absorbed at any other wavelength out of the above range.

This prevents the marks from being accidentally visible on the manufactured article of clothing, due for example to ultraviolet radiation or to other components that in any case are present in the sun light, as well as in the light emitted by normal artificial light sources, above all of the type used in particular places, such as laboratories, discotheques or others.

A possible absorption of this radiation emissions by the tracing substance could give rise to reflection or fluorescence phenomena that would make the marks undesirably visible on the article of garment worn. On the contrary, being the substance opaque in the infrared spectrum alone, this ensures that this substance cannot be seen by the human eye, not even under conditions of particular lighting.

Marking can be made more visible by use of a fluorescent tracing substance or in any case a substance capable of emitting the absorbed electromagnetic radiation again, preferably to a lower frequency than the absorbed radiation or in any case in the infrared spectrum, in such a manner as to prevent the emitted radiation from having frequencies falling within the visible spectrum (which on the contrary could happen with substances that are opaque to the ultraviolet radiation alone).

Use of fluorescent tracing substances as compared with merely opaque tracing substances allows much smaller amounts of substance to be used on the article of garment so that the possibility that some marks may be visible is made quite negligible, also in case of light-coloured surfaces.

In a preferred embodiment, the tracing substance comprises a liquid composition to be applied to the fabric by a suitable felt-tip pen or other writing instrument. This liquid composition can comprise the tracing substance in a solution.

If a cotton cloth is for example concerned, the solution is an aqueous solution, although it could also be an alcohol solution or involve still other solvents. In order to ensure adhesion of the substance to the fabric and enable it to be retained thereby, the substance can comprise functional groups adapted to bind the same to the fabric fibres.

Alternatively or in combination with the above and depending on the type of fabric on which marking is to be done, the aqueous composition may comprise a binding additive, such as an acrylic resin, to bind the substance to the fabric fibres.

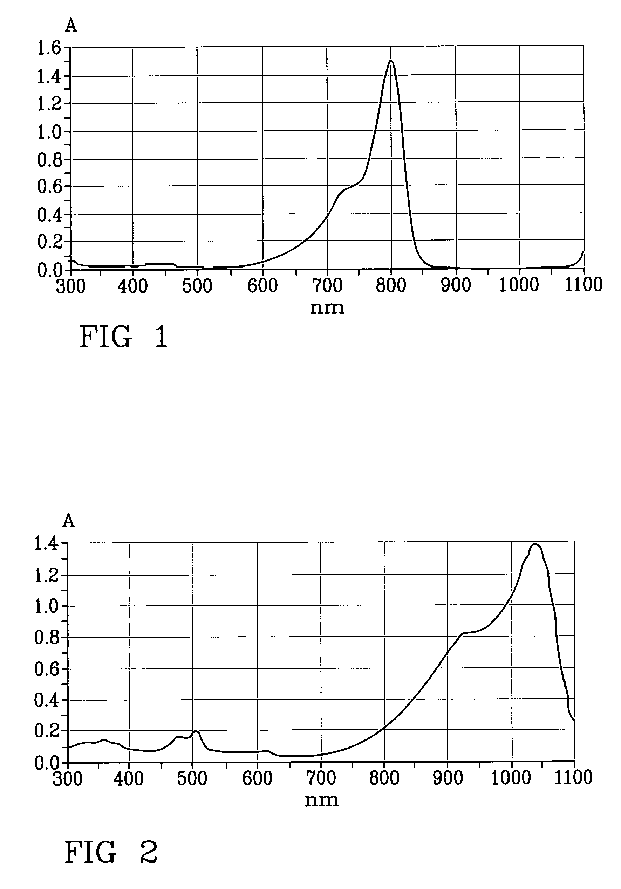

By way of example, the chemical formula A of a tracing substance adapted to be used in accordance with the present invention is reproduced hereinafter:
The graph in Fig. 1 shows the variation in the 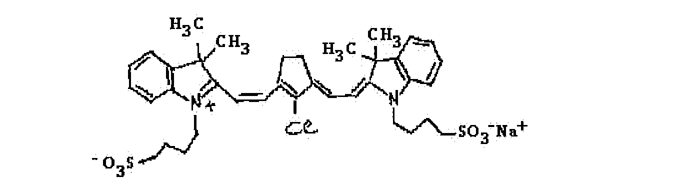 radiation absorption of such a substance, upon variation of the wavelength reproduced in the x-axes.
A further substance that can be usefully employed has the chemical formula B reproduced herebelow:
   The graph in Fig. 2 shows the variation in the 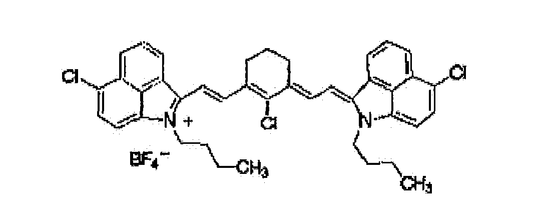 radiation absorption of such a substance, upon variation of the wavelength reproduced in the x-axes. This substance has an absorption peak with a radiation of a wavelength of 1035 nm and an emission peak at a wavelength of 1048 nm.

Another substance that can be also usefully employed has the following chemical formula C:

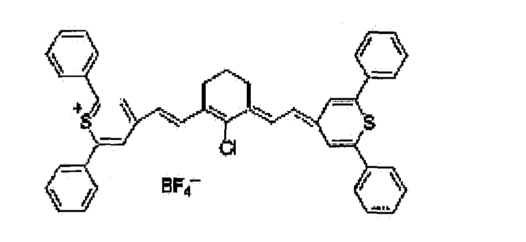

This substance has an absorption peak with a radiation of a wavelength of 1043 nm and an emission peak at a wavelength of 1058 nm.

The method of the present invention further contemplates detection of the marks on the textile product with a viewer sensitive to the electromagnetic radiation having wavelengths out of the visible spectrum, in particular wavelengths at which the tracing substance is opaque.

For instance, the viewer can comprise an image acquisition device, such as a digital video camera or photocamera possibly combined with a monitor for directly displaying the marks detected on the textile product.

Preferably a viewer should be used the sensitivity of which, upon variation of the wavelength of the radiation, has a peak and consequently a greater efficiency, at the wavelengths at which the tracing substance emits electromagnetic radiation. During the step of detecting the marks, the preferred embodiment of the invention further contemplates that the fabric be illuminated by a radiant source emitting radiation within the opacity range of the tracing substance.

In particular, the radiant source emits electromagnetic radiation mainly with wavelengths that are coincident with the wavelengths at which the tracing substance has an absorption peak. In more detail, it is preferable using a radiant source that, at wavelengths at which the tracing substance is opaque, is capable of emitting electromagnetic radiation in greater quantity than that of the radiation possibly emitted at wavelengths at which the tracing substance is transparent. This ensures excitation or at all events suitable illumination of the tracing substance, in such a manner that the same can be seen by the viewer to a maximum degree, relative to the surrounding material.

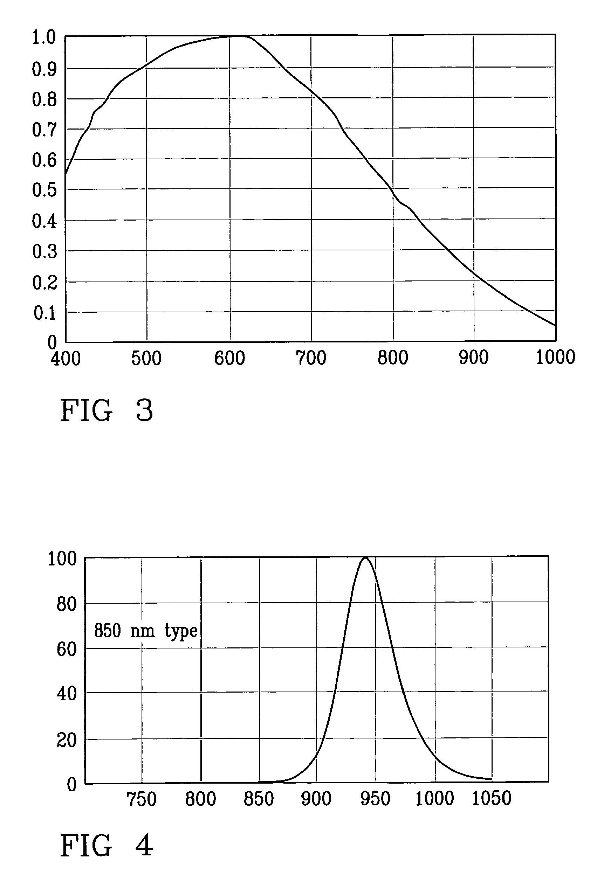

By way of example, a tracing substance of the type identified above in formula "A" can be better seen by illuminating the article of clothing with a radiant source having an emission peak for wavelength values in the order of 800 nm, and using a video camera having a sensitivity peak for said values. This does not eliminate the possibility of also using image acquisition devices and/or lighting devices having different features from those specified above, provided they are compatible with the absorption features of the tracing substance used. For instance, experimental tests with satisfactory results were carried out using, for tracing substances of the type corresponding to formulas "A" and "B", a video camera Guppy F-038B NIR by Allied Vision Technologies - Stadtroda (Germany) the sensitivity features of which are reproduced in the graph in Fig. 3, in combination with a LED radiant source with maximum emission at 940 nm produced by CCS-Inc of Kyoto (Japan), the relative emission intensity of which is represented by the graph in Fig. 4. The above graphs represent the sensitivity (Fig. 1) or emission (Fig. 2) variation in percentage relative to a peak value, depending on the values of the wavelength reproduced in the x-axes.

Using the combination of a fluorescent tracing substance in the infrared spectrum, a radiant source capable of only radiating in a narrow wavelength range corresponding to the maximum absorption of the tracing substance, or to the only wavelength corresponding to or close to said maximum absorption, and a viewer sensitive to the only wavelength of the radiation re-emitted by the tracing substance or having its maximum sensitivity in a narrow wavelength range close to or comprising said re-emitted radiation, any problem due to possible interferences connected with a radiation absorption by colouring substances or other chemical substances or by the fibres themselves (which possibility can never be completely excluded due to the great variety of chemical substances used in the industrial process) is advantageously eliminated. A tracing substance that is only opaque, on the contrary, could be easily "darkened" during the detection step, due to said interferences. The detection step further comprises acquisition through the viewer of a preferably digital image representative of the defects present on the fabric, to make this image available for electronic mapping of the fabric.

This electronic mapping of the fabric can be then stored in an electronic file to make it available during subsequent working steps of the fabric.

In this way, the data-containing file can be advantageously transmitted to the other operating seats and kept for subsequent controls.

Availability of this electronic mapping allows a preliminary remote visual and detailed examination of the faultiness conditions of the fabric, which involves time saving and does not require the material to be physically transferred, should unacceptable faultiness conditions be present.

In addition, the electronic mapping allows the information concerning the defects to be interfaced with a CAD (Computer-Aided Design) system for correct positioning, thus minimising the scraps during the fabric cutting step.

The invention achieves the intended purposes.

In fact, the mapping method of the present invention prevents possible disputes about the lack of announcement of defects due to causes that cannot be ascribed to the staff entrusted with the task of signalling the defects, because the marks cannot be accidentally removed or taken away, during transport or handling of the textile product for example.

In addition, the method of tracing defects of the present invention is not itself the cause of any defect to the fabric because the marks are invisible with the naked eye.

Finally, marking enables checks to be carried out, at any time and at any working stage of the marked fabric, on whether fabrics considered as unsuitable have been used for manufacturing garments.

Furthermore, being the marks invisible to the wavelengths at which the human eye is sensitive, articles carrying this marks can be used just the same, where the defect has been signalled by mistake or is however acceptable, as in the case of second-best articles of garment, without being obliged to resort to expensive and not always easy operations for removal of the marks.

## Claims

1. A method for tracing defects in textiles, comprising the steps of:
- detecting defects in a textile;
- making marks on said textile for identification of the detected defects;
**characterised in that** said marks are made by laying a tracing substance on the textile product, which substance is substantially transparent to electromagnetic radiation having wavelengths included in the visible spectrum and opaque to electromagnetic radiation having wavelengths out of the visible spectrum, and **in that** it comprises the step of detecting the marks on the textile by means of a viewer sensitive to electromagnetic radiation having wavelengths out of the visible spectrum.

2. A method as claimed in claim 1, wherein said tracing substance constituting the marks is opaque to radiation having wavelengths included in the infrared spectrum, preferably in the range of 770 nm to 1200 nm.

3. A method as claimed in claim 2, wherein said tracing substance re-emits the electromagnetic radiation absorbed; said re-emitted radiation having a wavelength in the range of the infrared.

4. A method as claimed in claim 3, wherein said re-emitted radiation has a lower frequency than that of the absorbed radiation.

5. A method as claimed in claim 2, wherein said tracing substance is a fluorescent substance.

6. A method as claimed in anyone of the preceding claims, comprising the step of illuminating the textile product with a radiant source emitting electromagnetic radiation during detection of the marks by means of said viewer.

7. A method as claimed in claim 1, wherein said viewer is sensitive to radiation having wavelengths relative to which said tracing substance is opaque.

8. A method as claimed in claim 1, wherein said viewer has such a sensitivity that, on varying of the wavelength of the detected variation, has a peak at the wavelengths relative to which said tracing substance is opaque.

9. A method as claimed in claims 3 and 8, wherein said viewer is sensitive to radiation having wavelengths included in the infrared spectrum, preferably in the range of 770 nm to 1200 nm.

10. A method as claimed in claim 6, wherein said source emits radiation having wavelengths relative to which said tracing substance is opaque.

11. A method as claimed in claim 6, wherein said radiant source at wavelengths at which the tracing substance is opaque, emits electromagnetic radiation to a greater amount as compared with that of the radiation emitted at wavelengths to which the tracing substance is transparent.

12. A method as claimed in claim 9, wherein said source emits radiation mainly having a wavelength included in the infrared spectrum, preferably in the range of 770 nm to 1200 nm.

13. A method as claimed in anyone of the preceding claims, wherein said tracing substance is a liquid composition and comprises functional groups adapted to bind said tracing substance to the fibres of said textile product.

14. A method as claimed in anyone of claims 1 to 12, wherein said tracing substance is a liquid composition comprising a binding additive for binding said tracing substance to the fibres of said textile product.

15. A method as claimed in claim 1, wherein said tracing substance has an electromagnetic-radiation absorption at a wavelength out of the visible spectrum at least 2.5 times greater than the electromagnetic-radiation absorption having a wavelength included in the visible spectrum.

16. A method as claimed in claim 1, wherein said step of detecting the defects comprises the step of acquiring, through said viewer, an image representative of the defects present on the fabric, to make this image available for electronic mapping of the fabric.

17. A method as claimed in claim 16, further comprising the step of storing the acquired image, to make said electronic mapping of the fabric available during subsequent working steps of the fabric.

18. A textile article of manufacture comprising at least one mark for identification of a defect, made in accordance with one or more of the preceding claims.
